(19) Europäisches Patentamt
European Patent Office
Office européen des brevets

(11) **EP 3 913 422 A1**

(12) **EUROPEAN PATENT APPLICATION**

(43) Date of publication:
**24.11.2021 Bulletin 2021/47**

(51) Int Cl.:
**G02C 7/04** (2006.01)  **G02C 7/08** (2006.01)
**G02B 3/14** (2006.01)  **G02B 26/00** (2006.01)

(21) Application number: **21175484.1**

(22) Date of filing: **22.05.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.05.2020 DE 102020113832**

(71) Applicant: **Optotune AG**
**8953 Dietikon (CH)**

(72) Inventors:
• ASCHWANDEN, Manuel
  6319 Allenwinden (CH)
• NIEDERER, David Andreas
  5024 Küttigen (CH)

(74) Representative: **Schulz Junghans**
**Patentanwälte PartGmbB**
**Großbeerenstraße 71**
**10963 Berlin (DE)**

(54) **CORRECTIVE LENS AND METHOD FOR FABRICATION OF A CORRECTIVE LENS**

(57)    Corrective lens comprising a carrier (1), a tuning element (2) and an actuator (3), wherein
- the carrier (1) has a first main surface (11), wherein the first main surface (11) is curved in two directions which two directions extend orthogonally with respect to each other,
- the tuning element (2) comprises a first flexible membrane, wherein the first flexible membrane has a contact area which is extensively connected to the first main surface, and
- the actuator is arranged to alter the optical power of the tuning element, wherein the actuator comprises a displacement unit with an electro permanent magnet.

Fig. 1

**Description**

The present corrective lens comprises a carrier, a tuning element and an actuator. The corrective lens may be a contact lens which is arranged to be placed directly on the surface of the eyes. The corrective lens may be an intraocular lens, which is arranged to be implanted in the eye for example as part of a treatment for cataracts or myopia. Corrective lenses are ocular prosthetic devices which are arranged to correct vision for cosmetic or therapeutic reasons.

The carrier is transparent for visible light. In particular, the carrier comprises silicone and/or fluorocarbon. For example, the carrier has essentially the shape of a spherical segment, in particular the shape of a corrective lens. The carrier has a first main surface, wherein the first main surface is curved in two directions which two directions extend orthogonally with respect to each other. In particular, the first main surface of the carrier is concavely curved.

The carrier may have a transparent central region and an edge region, wherein the edge region surrounds the central region. The central region can be provided so that a user can see through it. The carrier may comprise an opening in the central region, wherein the tuning element is arranged to be at least partially inserted into said opening. The edge region may comprise opaque material. In particular, the edge region may comprise electronic components in the edge region.

The tuning element may be arranged to interact with visible light by means of refraction or diffraction. The interaction with visible light of the tuning element may be controlled and altered in operation of the tuning element. In particular, the tuning element is a tunable lens, a tunable grating or a tunable mirror. The tuning element comprises a first flexible membrane. The first flexible membrane may have a contact area which is extensively connected to the first main surface of the carrier. Here and in the following "extensively connected" describes a connection having a continuous contact area. In particular, said contact area continuously extends over a major part of the first main surface in the central region. In the contact area the first membrane rests against the first main surface and takes on the shape of the first main surface. In particular, the difference between the refractive index of the carrier and the refractive index of the first membrane is less than 0.2, preferably less than 0.1, highly preferred less than 0.05.

The actuator is arranged to control the tuning element. In particular, the actuator moves components of the tuning element with respect to the carrier, whereby the interaction of the tuning element with visible light is controlled. The actuator is arranged to alter the optical power of the tuning element. The actuator comprises a displacement unit which is arranged to exert a first force onto the tuning element. The displacement unit may comprise an electro permanent magnet. Alternatively, the displacement unit may comprise a piezo element. For example, the actuator comprises multiple displacement units, wherein each displacement unit is arranged to exert a first force onto the tuning element. The displacement units may be arranged in the edge region of the carrier. In particular, the displacement unit is attached to a circumferential portion of the carrier. According to one embodiment, the actuator comprises multiple displacement units, each having an electro permanent magnet, wherein the displacement units are arranged around the tuning element on the carrier.

The displacement unit comprises a at least one permanent magnet. In this context, a permanent magnet is an object made from a material that is magnetized and creates its own persistent magnetic field. In particular, the permanent magnet comprises a ferromagnetic material.

According to one embodiment, the corrective lens comprises the carrier, the tuning element and the actuator. The carrier has a first main surface, wherein the first main surface is curved in two directions which two directions extend orthogonally with respect to each other.

The tuning element comprises the first flexible membrane. The first flexible membrane may have a contact area which is extensively connected to the first main surface. The actuator is arranged to alter the optical power of the tuning element, wherein the actuator comprises the displacement unit with an electro permanent magnet.

The corrective lens described is based, among other things, on the consideration that a fixed optical power limits the field of application of such a corrective lens. Thus, it is desired to be able to adapt the optical power in different situations to the needs of the user. Furthermore, it is desired, that the corrective lens is universally applicable to correct different ametropia. The present corrective lens advantageously overcomes said limitations by altering the optical power of the corrective lens by means of the tuning element.

According to one embodiment, the carrier comprises a second main surface on a side opposed to the first main surface. In particular, the second main surface is convexly curved. For example, the second main surface extends essentially parallel to the first main surface.

According to one embodiment the at least one vent extends from the first main surface to the second main surface through the carrier. The vent may be an opening which allows gas exchange, in particular oxygen exchange, between the side of the first main surface and the side of second main surface.

According to one embodiment, the tuning element comprises a cavity which is filled with a liquid. In particular, the liquid is transparent for visible light.

According to one embodiment the tuning element comprises a second flexible membrane and a shaper. The second flexible membrane may consist of the same material as the first flexible membrane. In particular, the first flexible membrane and the second flexible membrane may be fabricated in a one-piece manner. For example, the first and the second flexible membrane surround the cavity which is filled with the liquid completely on all sides. Alternatively, the cavity is limited by the first flexible membrane, the second flexible membrane and the shaper. In particular, the shaper may have a frame like geometry, wherein the first and the second membrane are fixedly attached to opposing sides of the shaper.

The actuator is arranged to adjust a position of the shaper with respect to the carrier. The second flexible membrane depends on the position of the shaper with respect to the carrier. In particular, a reduced distance between the shaper and the carrier results in an increased optical power and an increased distance between the shaper and the carrier results in a reduced optical power.

According to one embodiment, the actuator is arranged to apply a first force to the shaper in a first direction and a returning structure is arranged to apply a second force to the shaper in a second direction. The first direction and the second direction are in opposite directions. In particular, the first force is an adjustable magnetic force and the second force is a spring force. The returning structure may be fabricated in a one-piece manner with the shaper. In particular, the returning structure may be formed as a bending beam, which is fixedly attached to the carrier.

According to one embodiment, the displacement unit comprises a first permanent magnet, a second permanent magnet, a coil and two pole pieces. The coil is arranged to adjust the intensity and direction of the magnetization of the first permanent magnet. In particular, the first permanent magnet is surrounded by the coil, whereby the first permanent magnet is exposed to magnetic fields, which result from currents through the coil. In particular, also the second permanent magnet is arranged in the coil. For example, the first permanent magnet and the second permanent magnet may comprise materials having a different susceptibility. For example, the susceptibility of the first permanent magnet is larger than the susceptibility of the second permanent magnet. Thus, the exposure of the first and the second magnet to the same magnetic field results in different magnetizations of the first and the second permanent magnet.

In particular, the first and the second permanent magnet have each a main extension axis, wherein the main extension axis of the first permanent magnet extends parallel to the main extension axis of the second magnet. In a direction obliquely to the main extension axes, both magnets may be surrounded by the coil. In particular, the coil comprises a single winding.

The pole pieces are arranged on opposing sides of the of the first and second permanent magnet along their main extension axes. In particular, each pole piece covers the end of the south pole or the north pole of the first and the second permanent magnet. The pole pieces comprise a ferromagnetic material, in particular iron. The pole pieces are arranged, to conduct the magnetic field of the first and second permanent magnet. The pole pieces are arranged in close proximity to the shaper. In particular, the shaper comprises a material having a susceptibility not equal to zero. For example, the susceptibility of the material of the shaper is larger than 100, in particular larger than 200, preferably larger than 300.

By applying a current to the coil, the magnetization in the first and second permanent magnet may be altered, whereby the displacement unit may be altered into different states. In a first state, the magnetization of the first permanent magnet is anti-parallel to the magnetization of the second permanent magnet. Thus, the magnetization of the first permanent magnet counteracts the magnetization of the second permanent magnet. Hence, in the first state, the displacement unit does not affect a magnetic force onto the shaper. In a second state, the magnetization of the first permanent magnet is parallel to the magnetization of the second permanent magnet. Thus, the magnetization of the first permanent magnet and the second permanent magnet sum up. In the second state, the first and second permanent magnet, the pole pieces and at least a section of the shaper from a magnetic circuit, whereby a magnetic force exerts onto the shaper.

According to one embodiment, the first flexible membrane has a contact area which is extensively connected to the first main surface. In particular, the carrier comprises a circumferential portion and a central portion. The central portion is arranged in an opening extending completely through the circumferential portion. The central portion comprises the first main surface. In particular, when fabricating the corrective lens, the first membrane is attached to the first main surface of the central portion and afterwards the central portion is attached to the circumferential portion.

A method for fabrication of a corrective lens is also specified. In particular, a corrective lens described here can be produced with the method. This means that all of the features disclosed for the corrective lens are also disclosed for the method and vice versa.

In a method step a) of the method for fabrication of a corrective lens a carrier with a first main surface is provided, a tuning element is provided with a first flexible membrane and second flexible membrane on opposing sides of the tuning element, and an actuator is provided.

In a method step b) of the method for fabrication of a corrective lens the tuning element is attached to the carrier. For example, the first flexible membrane and the first main surface may be adhesively connected.

In a method step c) the actuator is arranged on the carrier. In particular method step c) may be performed between method step a) and method step b).

According to one embodiment, in method step b) the second flexible membrane is attached to a stamping tool. The stamping tool pushes the tuning element against the carrier, wherein the first flexible membrane and the first main surface form a contact area. In particular, the stamping tool is arranged to push the tuning element against the first main surface of the carrier. After connecting the tuning element and the carrier, the stamping tool is removed.

According to one embodiment of the method, in step a) the first flexible membrane extends essentially flat and in step b) the first flexible membrane is curved by means of the stamping tool and by means of the first main surface of the carrier.

According to one embodiment the stamping tool comprises a stamping surface, which is essentially complementary to the first main surface. For example, the stamping surface is at least in a section convexly curved. In method step b) the tuning element is attached to the stamping surface with the second membrane. The second membrane rests against the stamping surface, whereby the second membrane and the first membrane are curved.

According to one embodiment, the carrier comprises a vent in the first main surface, wherein in method step b) a fluid between the first flexible membrane and the first main surface is removed through the vent. The vent may extend continuously from the first main surface to the second main surface. The fluid may be gaseous, like air, or liquid, like oil, water of an adhesive.

Further advantages and advantageous refinements and developments of the corrective lens and the method for fabrication of the corrective lens result from the following exemplary embodiments illustrated in connection with the figures.

Figures 1 and 2 show exemplary embodiments of corrective lenses in schematic perspective views;

Figures 3 and 4 show exemplary embodiments of corrective lenses in schematic section views;

Figure 5 shows a tuning element of an exemplary embodiment of the corrective lens in a schematic perspective view which is provided in method step a) of the method for fabrication of a corrective lens;

Figure 6 shows a carrier and an actuator of an exemplary embodiment of a corrective lens in a schematic section view which are provided in method step a) of the method for fabrication of a corrective lens;

Figure 7 shows an exemplary embodiment of method step b) of the method for fabrication of the corrective lens in a schematic section view, wherein the tuning element is attached to the carrier by means of a stamping tool;

Figure 8 shows an exemplary embodiment of a displacement unit of a corrective lens in a schematic perspective view;

Figure 9, 10 and 11 show exemplary embodiments parts of a displacement unit of a corrective lens in schematic perspective views;

Figure 12, 13 and 14 show exemplary embodiments of parts of a displacement unit of a corrective lens in schematic section views;

Figure 15 and 16 show an exemplary embodiment of a tuning element of a corrective lens in a top view and in a section view;

Figure 17 shows an exemplary diagram of a tuning element, which displays the relation of first force applied to the tuning element by means of the actuator and optical power of the tuning element;

Figure 18 shows an exemplary diagram of a tuning element, which displays the relation of shaper stroke to optical power;

Figure 19 shows an exemplary diagram of an actuator, which displays the relation of a first force to the length of the gap between the actuator and the shaper;

Figure 20 shows an exemplary diagram of an actuator, which displays the relation of the current applied to the coil to the air gap between the actuator and the shaper;

Figure 21 shows of an exemplary embodiment of a corrective lens in a schematic perspective sectional view;

Figure 22 shows a portion of an exemplary embodiment of a corrective lens in a schematic perspective sectional view;

Figure 23 shows an exemplary embodiment of corrective lens in a schematic perspective sectional view and two enlarged sectional views of the exemplary embodiment.

Identical, identical or identically acting elements are provided with the same reference symbols in the figures. The figures and the proportions of the elements shown in the figures among one another are not to be regarded as to scale, unless units are expressly stated. Rather, individual elements may be exaggerated in size for better displayability and/or for better comprehensibility.

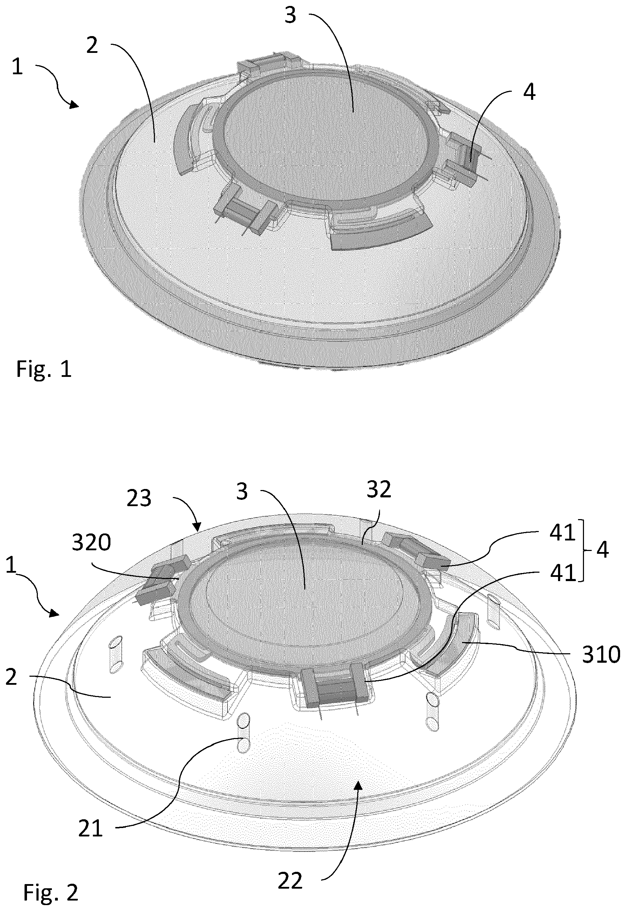

Figures 1 and 2 show exemplary embodiments of corrective lenses in schematic perspective views. The corrective lens 1 comprises a carrier 2, a tuning element 3 and an actuator 4.

The carrier 2 has a first main surface 22, which is concavely curved, and a second main surface 23, which convexly curved. The carrier 1 has a first main surface 22, wherein the first main surface 22 is curved in two directions which two directions extend orthogonally with respect to each other. On the side of the first main surface 22, the tuning element 3 and the actuator 4 is attached to the carrier 2. The carrier 2 comprises vents 21, which enable gas exchange between the side of the first main surface 22 and the second main surface 23.

The actuator 4 comprises multiple displacement units 41, wherein each displacement unit is arranged to exert a first force 61, in particular a magnetic force, onto the tuning element 3, in particular onto a section 320 of a shaper 32. The tuning element 3 comprises a returning structure 310, which exerts a second force 62, in particular a returning force, in direction against the magnetic force.

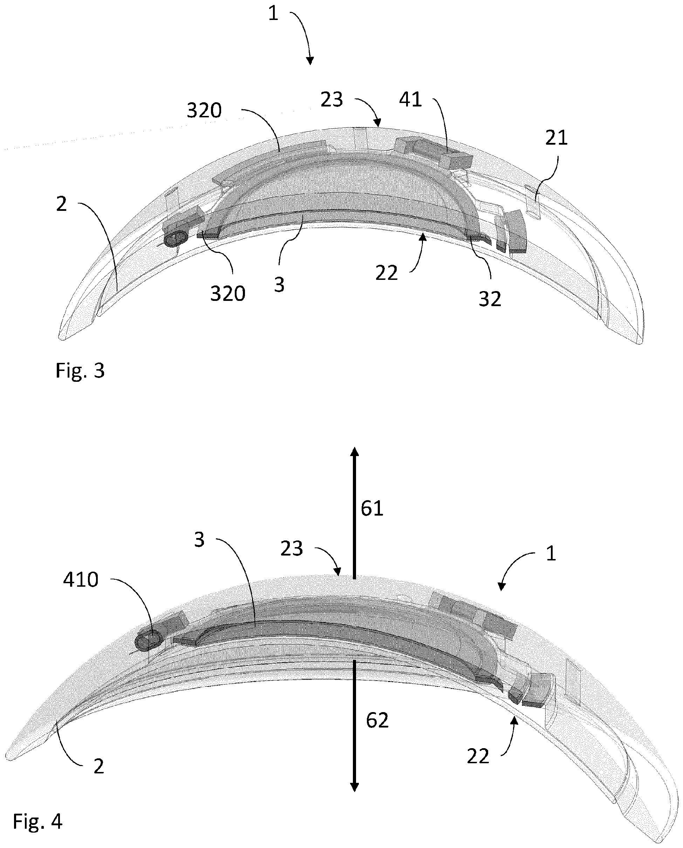

Figures 3 and 4 show exemplary embodiments of corrective lenses 1 in schematic section views. The tuning element 2 comprises a first flexible membrane 33, wherein the first flexible membrane 33 has a contact area which is extensively connected to the first main surface 22. The actuator 4 is arranged to alter the optical power of the tuning element 3, wherein the actuator 4 comprises a displacement unit 41 with an electro permanent magnet 410.

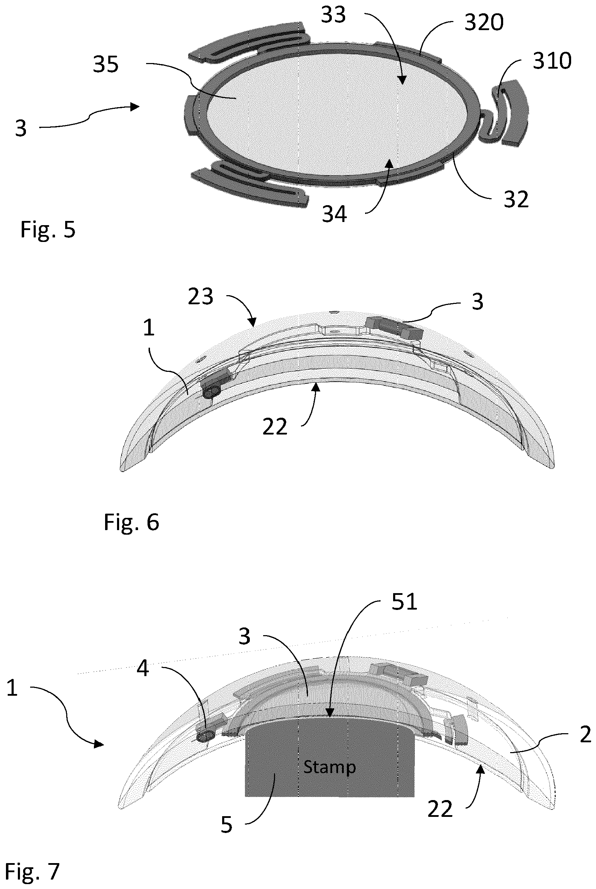

Figure 5 shows a tuning element 3 of an exemplary embodiment of the corrective lens 1 in a schematic perspective view. The tuning element 3 is provided in method step a) of the method for fabrication of a corrective lens 1. The tuning element comprises a shaper 32 which has the geometry of a circular frame. Additionally, the shaper 32 comprises the first flexible membrane 33 and the second flexible membrane 34, which are arranged on opposing sides of the shaper 32. The shaper 32, the first flexible membrane 33 and the second flexile membrane 34 enclose a cavity, which is filled with a transparent liquid 35. The shaper 32 is made of a ferromagnetic material, which is arranged to interact with the actuator 4. The shaper comprises multiple sections 320, which are arranged circumferentially around the cavity. The shaper 32 comprises multiple returning structures, which are arranged circumferentially around the cavity. The returning structures 310 are embodied as meandering bending beams. The distal ends of the bending beams are arranged to be attached to the carrier 2.

Figure 6 shows a carrier 2 and displacement units 41 of an actuator 4 of an exemplary embodiment of a corrective lens 1 in a schematic section view. The carrier 2 and the actuator are provided in method step a) of the method for fabrication of a corrective lens 1. The displacement units 41 are attached to the carrier 2 in method step c) of the method for fabrication of a corrective lens 1.

Figure 7 shows an exemplary embodiment of method step b) of the method for fabrication of the corrective lens 1 in a schematic section view. The tuning element 3 is attached to the carrier 2 by means of a stamping tool 5. The stamping tool 5 has a stamping surface 51, which is convexly curved. The stamping surface 51 pushes the tuning element 3 against the first main surface 22 of the carrier 2. Thereby the tuning element 3, in particular the first flexible membrane 33 is curved. In particular, the first flexible membrane 33 and the first main surface 22 are connected to each other in a contact area. The contact area covers at least 75%, in particular at least 90%, of the surface of the first flexible membrane 33 facing the first main surface 22. The stamping tool 5 is removed after method step b).

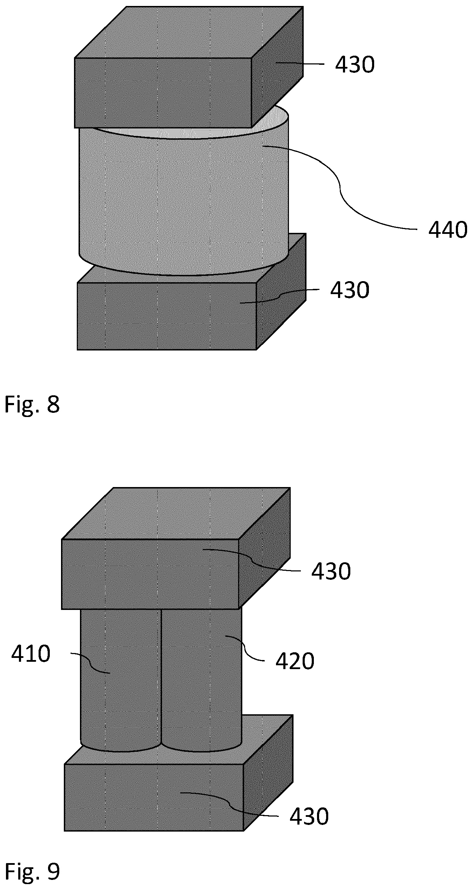

Figures 8 and 9 show an exemplary embodiment of a displacement unit 41 of a corrective lens 1 in schematic perspective views. The displacement unit 41 comprises two pole pieces 430 and a coil 440. The coil 440 surrounds a first permanent magnet 410 and a second permanent magnet 420. The susceptibility of the first permanent magnet is larger than the susceptibility of the second permanent magnet 420. For example, the first permanent magnet comprises iron, aluminum, nickel cobalt (Alnico) and the second permanent magnet comprises nickel, iron and boron (NIB).

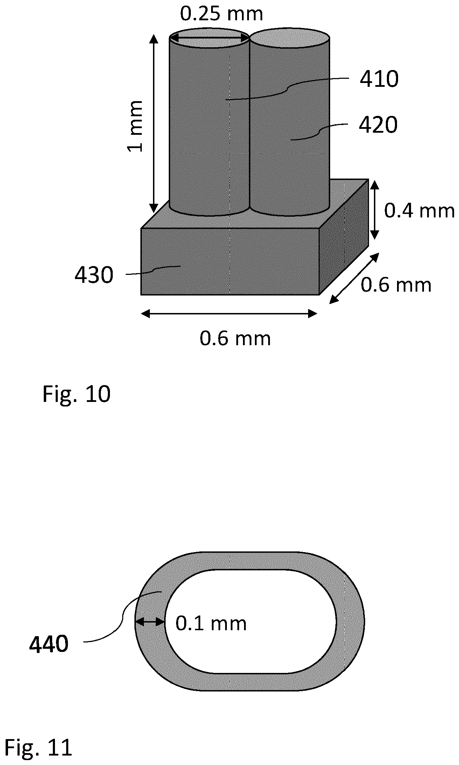

Figure 10 and 11 show parts of a displacement unit 41 of a corrective lens 1 and the dimensions of such exemplary embodiment. The first 410 and the second 420 permanent magnet have a cylindrical geometry, having a diameter of 0.25 mm and a length of 1 mm. The pole piece 430 has a cuboid shape with 0.6 mm X 0.6 mm X 0.4 mm. The coil 440 has a single winding, wherein the thickness of the coil material is 0.1 mm.

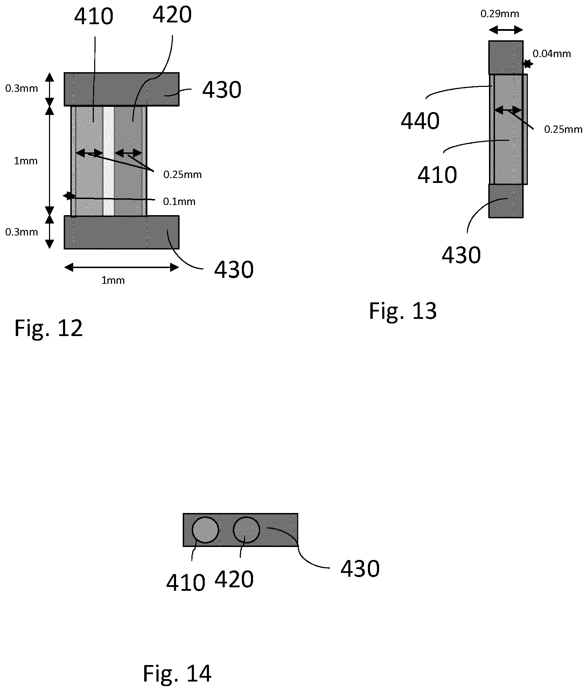

Figure 12, 13 and 14 show exemplary embodiments of parts of a displacement unit 41 of a corrective lens 1 in schematic section views. The dimensions of the pole pieces 430 derive slightly from the dimensions of the embodiment shown in figures 10 and 11.

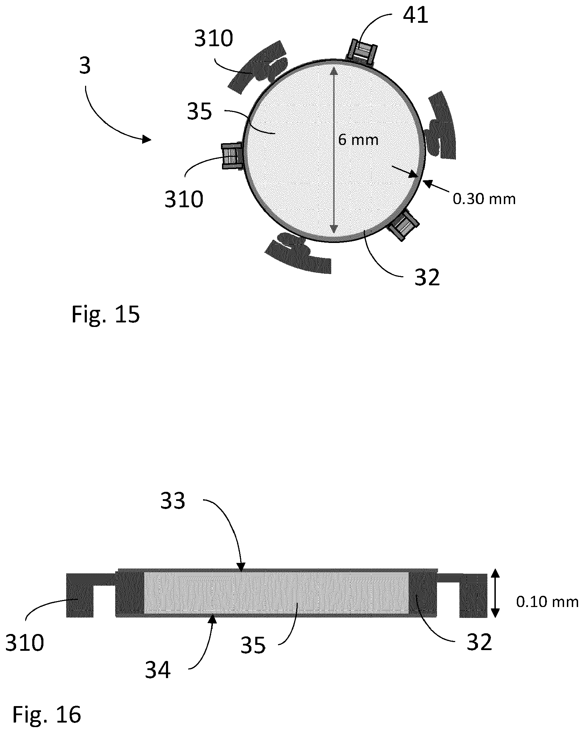

Figure 15 and 16 show an exemplary embodiment of a tuning element of a corrective lens in a top view and in a section view. The tuning element 3 has a clear aperture with a diameter of 6 mm. The shaper 32 has a width of 0.3 mm. The gap between the displacement unit and the sections, measured along the optical axis, is approximately 20 micrometers. The shaper 32 comprises multiple sections 310, wherein a displacement unit 410 is assigned to each section 310. Furthermore, the shaper comprises multiple returning structures 310. The shaper 32 has a thickness of 0.1 mm. The thickness is measured along the optical axis and the width is measured perpendicular to the optical axis. On opposing sides of the shaper 32 the first flexible membrane 33 and the second flexible membrane 34 are arranged. The flexible membranes each have a thickness of 40 micrometer. The first flexible membrane 33, the second flexible membrane 34 and the shaper 32 limit a cavity, which is completely filled with the transparent fluid 35.

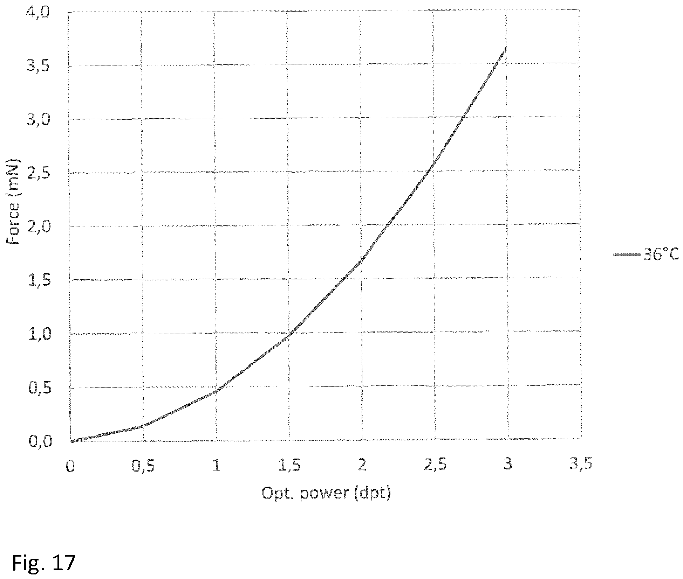

Figure 17 shows an exemplary diagram of a tuning element 3, which displays the relation of force applied to the tuning element 3 by means of the actuator 4 and the optical power of the tuning element. The optical power is given in diopters, the force is given in Millinewton. The data is provided for ambient temperature of 36°C.

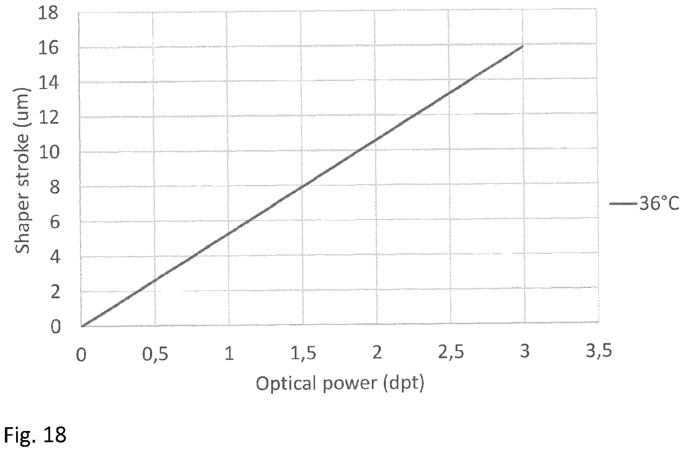

Figure 18 shows an exemplary diagram of a tuning element 30, which displays the relation of shaper stroke to optical power. The optical power is given in diopters, the shaper stroke is given in micrometers. The data is provided for an ambient temperature of 36°C.

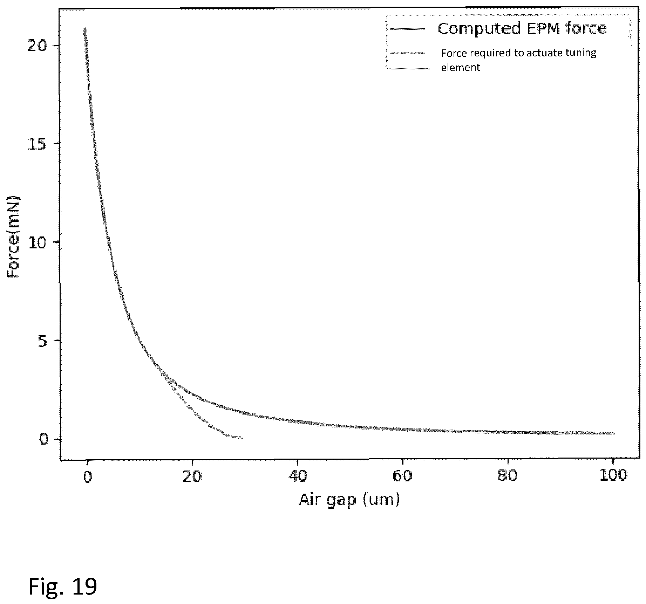

Figure 19 shows an exemplary diagram of an actuator, which displays the relation of the force to the length of the gap between the actuator and the shaper. The force is given in millinewton and the length of the air gap is given in micrometer. The blue curve shows the force of the actuator 4 and the orange curve shows the force of the tuning element 3. An analytical magnetic circuit model is used to estimate the force of the actuator. The force of the actuator (blue curve) is compared to the force of the tuning element 3.

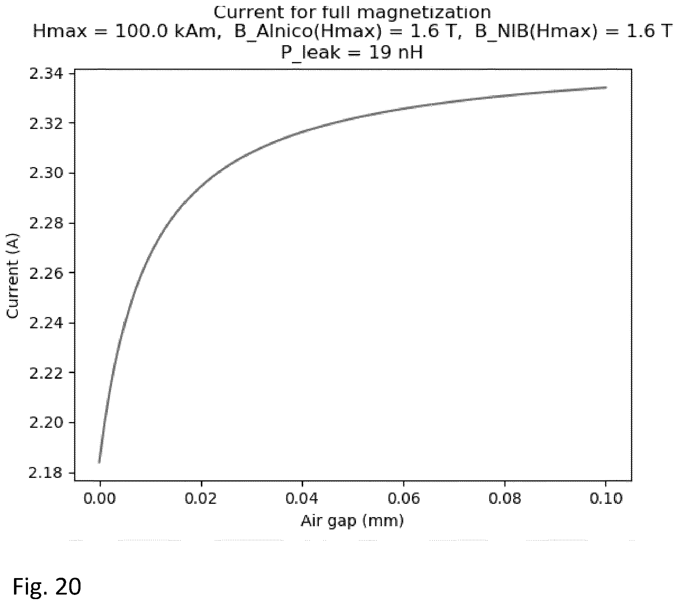

Figure 20 shows an exemplary diagram of an actuator, which displays the relation of the current applied to the coil to the length of the gap between the actuator and the shaper. The current is given in ampere and the length of the gap is given in millimeter. For example, for a coil built with a 40 micrometers diameter wire having 50 windings and 1 ohm resistance the actuator may be driven with a current of less than 5 amperes, in particular less than 2.5 ampere. Said current requires a voltage between +5 volts and -5 volts, in particular between -2.5 volts and 2.5 volts.

The actuator requires a maximum power for switching between two states of the actuator of 1 millijoule, preferably of 0.625 millijoule, when the switching time (t) is 100 micro seconds, the resistance is 1 ohm, the driving voltage (U) is 2 volts and the driving current (I) is 2.5 ampere. The energy consumption (E) per switching is calculated by:

$$E = P * t = U * I * t$$

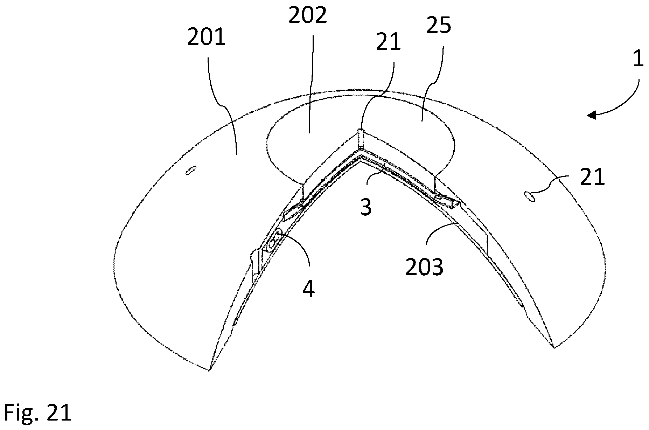

Figure 21 shows of an exemplary embodiment of a corrective lens 1 in a schematic perspective sectional view with a 90° section removed. The corrective lens may be a contact lens or an intraocular lens. The carrier 2 comprises a circumferential portion 20, a central portion 202 and a back cover 203. In particular, except for the arrangement of vents 21 in the carrier, the carrier 2 is essentially rotationally symmetric. The carrier 2 has the shape of a meniscus lens, wherein a first surface has a concave shape and a second surface has a convex shape. The circumferential portion 201 has an opening extending from the first surface to the second surface completely through the circumferential portion 201. The circumferential portion 201 surrounds the opening in one plane completely. The circumferential portion has a recess which is arranged adjacent to the opening. The recess forms a step, wherein the step extends around the opening.

The central portion 202 is arranged in the opening. A surface of the central portion and a surface of the circumferential portion together form the second surface of the carrier. The central portion 202 is form fittingly arranged in the opening of the circumferential portion.

The back cover is arranged in the recess of the circumferential portion 201. The back cover forms at least a portion of the first main surface of the carrier 2. The central portion 202, the circumferential portion 201 and the back cover 203 delimit a cavity. The actuator 4 and the tuning element 3 are arranged in the cavity. The actuator 4 may be comprise a piezo element and/or electropermanent magnets for adjusting optical properties of the tuning element 3.

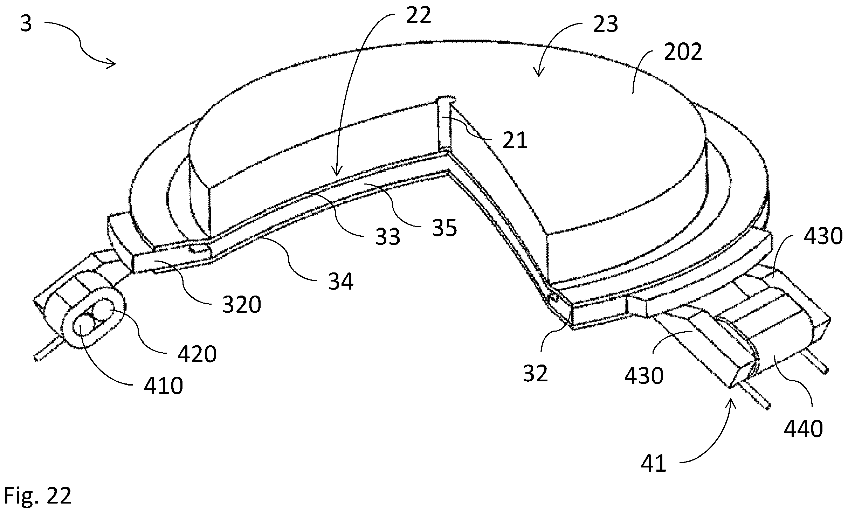

The circumferential portion comprises vents 21, which are arranged to enable gas exchange between the first and the second surface of the carrier 2. The central portion comprises a vent 21, which completely extends through the central portion 202. The vent 21 in the central portion 202 is utilized for fabrication purposes as described in connection with figure 22.

Figure 22 shows a portion of an exemplary embodiment of a corrective lens 1 in a schematic perspective sectional view. Compared to the embodiment in figure 21, the circumferential portion 201 and the back cover 203 are not depicted. The tuning element 3 comprises a first and a second flexible membrane 33, 34 having a transparent liquid 35 in between. The first and the second membrane 33,34 form refractive surfaces, wherein the optical properties of the tuning element 3 are adjusted by adjusting the shape of the first and/or second membrane 33, 34.

The method for fabricating the corrective lens 1 comprises a method step a) in which the carrier 2, in particular the central portion 202, with a first main surface 22 is provided. The tuning element 3 with the first flexible membrane 33 and the second flexible membrane 34 on opposing sides of the tuning element 3 are provided, and the actuator 4 is provided.

In a method step b) the tuning element 3 is attached to the carrier 2, wherein a fluid between the first flexible membrane 34 and the first main surface is removed through the vent 21. The fluid maybe a gas, in particular air, or a liquid, in particular oil, water or an adhesive. According to a first alternative, a negative pressure may be applied through the vent 21, wherein the first membrane has initial contact to the carrier in the region of the vent 21. The contact area between the first membrane and the carrier 2 increases starting from the region of the vent, wherein the contact area is a connected area, in particular a simply connected area, in a mathematical sense. In particular, the negative pressure affixes the first membrane 33 to the carrier 2 during fabrication. According to a second alternative the first membrane has initial contact to the carrier in a region of the outer rim of the central portion 202. The contact area between the first membrane 33 and the central portion 202 increases from the outer rim towards the vent 21.

In a method step c) the actuator 4 is arranged on the carrier 2. In particular, the actuator may be attached to the circumferential portion 201 after the central portion 202 with the tuning element 3 is attached to the circumferential portion 201.

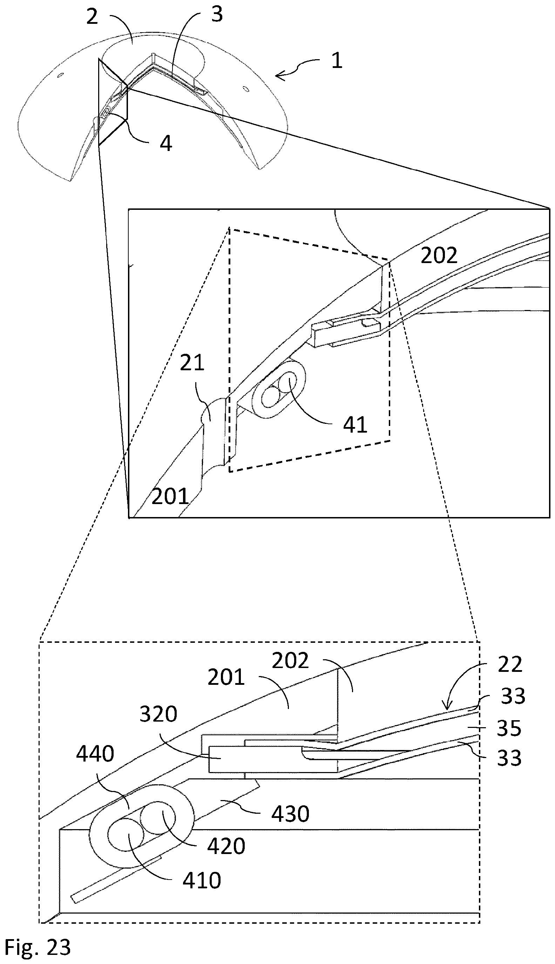

Figure 23 shows an exemplary embodiment of corrective lens 1 in a schematic perspective sectional view and two enlarged sectional views of the exemplary embodiment.

The schematic perspective sectional view displays the same embodiment as figure 21. The first enlarged sectional view, which is framed with a continuous line, shows details of the carrier 2, the actuator 4 and the tuning element 3. In the first enlarged sectional view, the representation is simplified by omitting the back cover 203. The circumferential portion 201 of the carrier 2 comprises a vent extending from the first surface to the second surface of the carrier, wherein the vent 21 enables gas exchange. The actuator 4 comprises a displacement unit 41, which is arranged to move the shaper 32 by applying a force to the section 320.

The second enlarged sectional view, which is framed with a dashed line, shows details of the carrier 2, the actuator 4 and the tuning element 3. In the second enlarged sectional view, the representation is simplified by omitting the back cover 203. The displacement unit 41 comprises a first permanent magnet 410, a second permanent magnet 320, a pole piece 430 and a coil 440. The magnetization of the first and second permanent magnets is adjustable by applying a current to the coil 440. The magnetization results in a force, which moves the section 320. The movement of the section 320 results in a displacement of the shaper 32, whereby the curvature of the second membrane 34 is adjusted. In particular, the shaper 32, the liquid 35 and the second membrane 34 are suspended solely by means of the first membrane 33. Alternatively, the shaper 32 comprises a returning structure 310, wherein the shaper 32, the liquid 35 and the second membrane 34 are also suspended by means of the returning structure 310.

The invention is not restricted to the exemplary embodiments by the description based on these. Rather, the invention encompasses every new feature and every combination of features, which in particular includes every combination of features in the patent claims, even if this feature or this combination itself is not explicitly specified in the patent claims or exemplary embodiments.

List of reference signs

| 1 | Corrective lens |
| 2 | Carrier |
| 21 | Vent |
| 22 | First main surface |
| 23 | Second main surface |
| 201 | Circumferential portion |
| 202 | Central portion |
| 203 | Back cover |
| 3 | Tuning element |
| 310 | Returning structure |
| 32 | Shaper |
| 320 | Section |
| 33 | First flexible membrane |
| 34 | Second flexible membrane |
| 35 | Transparent liquid |
| 4 | Actuator |
| 41 | Displacement unit |
| 410 | First permanent magnet |
| 420 | Second permanent magnet |
| 430 | Pole piece |
| 440 | Coil |
| 450 | Piezo element |
| 5 | Stamping tool |
| 51 | Stamping surface |
| 61 | First force |
| 62 | Second force |

**Claims**

1. Corrective lens (1) comprising a carrier (2), a tuning element (3) and an actuator (4), wherein

   - the carrier (2) has a first main surface (22), wherein the first main surface (22) is curved in two directions which two directions extend orthogonally with respect to each other,
   - the tuning element (3) comprises a first flexible membrane (33), and
   - the actuator (4) is arranged to alter the optical power of the tuning element (3), wherein the actuator (4) comprises a displacement unit (41) with an electro permanent magnet (410, 420) or with a piezo element (450).

2. Corrective lens (1) according to claim 1, wherein the curvature of the first main surface (22) is concave.

3. Corrective lens (1) according to one of the preceding claims, wherein
   the carrier (2) comprises a second main surface (23) on a side opposed to the first main surface (22), and the second main surface (23) extends essentially parallel to the first main surface (22).

4. Corrective lens (1) according to the preceding claim, wherein the at least one vent (21) extends from the first main surface (22) to the second main surface (23) through the carrier (2).

5. Corrective lens (1) according to one of the preceding claims, wherein
   the tuning element (3) comprises a cavity which is filled with a transparent liquid (35).

6. Corrective lens (1) according to the preceding claim, wherein

- the tuning element (3) comprises a second flexible membrane (34) and a shaper (32),
- the actuator (4) is arranged to adjust a position of the shaper (32) with respect to the carrier (2), and
- a curvature of the second flexible membrane (34) depends on the position of the shaper (32) with respect to the carrier (2).

7. Corrective lens (1) according to the preceding claim, wherein the first flexible membrane (33) and the second flexible membrane (34) limit the cavity on all sides completely.

8. Corrective lens (1) according to one of the preceding claims, wherein

- the actuator (4) comprises multiple displacement units (41), each having an electro permanent magnet (410, 420), and
- wherein the displacement units (41) are arranged around the tuning element (3) on the carrier (2).

9. Corrective lens (1) according to one of the preceding claims, wherein the actuator (4) is arranged to apply a first force (61) to the shaper (32) in a first direction and a returning structure (310) is arranged to apply a second force (62) to the shaper (32) in a second direction, wherein the second direction is anti-parallel to the first direction.

10. Corrective lens (1) according to the preceding claim, wherein the first force (61) is an adjustable magnetic force and the second force (62) is a spring force.

11. Corrective lens (1) according to one of the preceding claims, wherein the first flexible membrane (33) has a contact area which is extensively connected to the first main surface (22).

12. Method for fabrication of a Corrective lens (1), comprising the steps of:

a) providing

- a carrier (2) with a first main surface (22),
- a tuning element (3) with a first flexible membrane (33) and a second flexible membrane (34) on opposing sides of the tuning element (3), and
- an actuator (4),

b) attaching the tuning element (3) to the carrier (2), c) arranging the actuator (4) on the carrier (2).

13. Method according to claim 12, wherein in method step b)

- the second flexible membrane (34) is attached to a stamping tool (5),
- the stamping tool (5) pushes the tuning element (3) against the carrier (2), wherein the first flexible membrane (33) and the first main surface (22) form a contact area, and
- the stamping tool (5) is removed from the tuning element (3).

14. Method according to claim 12 or 13, wherein

- in step a) the first flexible membrane (33) extends essentially flat,
- in step b) the first flexible membrane (33) is curved by means of the first main surface (22) of the carrier (22).

15. Method according to claim 14, wherein the stamping tool (5) comprises a stamping surface (51), which is essentially complementary to the first main surface (22).

16. Method according to claim 12, wherein the carrier 2 comprises a vent (21) in the first main surface (22), wherein in method step b) a fluid between the first flexible membrane (34) and the first main surface is removed through the vent (21).

Fig. 1

Fig. 2

Fig. 3

Fig. 4

Fig. 5

Fig. 6

Fig. 7

430

440

430

Fig. 8

430

410

420

430

Fig. 9

0.25 mm

410

420

1 mm

0.4 mm

0.6 mm

430

0.6 mm

Fig. 10

440

0.1 mm

Fig. 11

Fig. 12

Fig. 13

Fig. 14

41

310

3

35

6 mm

310

0.30 mm

32

Fig. 15

33

310

0.10 mm

34     35     32

Fig. 16

Fig. 17

Fig. 18

Fig. 19

Current for full magnetization
Hmax = 100.0 kAm, B_Alnico(Hmax) = 1.6 T, B_NIB(Hmax) = 1.6 T
P_leak = 19 nH

Fig. 20

EP 3 913 422 A1

Fig. 21

Fig. 22

EP 3 913 422 A1

Fig. 23

Europäisches
Patentamt

European
Patent Office

Office européen
des brevets

**EUROPEAN SEARCH REPORT**

Application Number

EP 21 17 5484

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/028847 A1 (OPTOTUNE AG [CH]) 15 February 2018 (2018-02-15) | 1-3,5,8 | INV. G02C7/04 |
| Y | * page 15, line 3 - page 23, line 13; figures 1-14 * | 1-16 | G02C7/08 G02B3/14 G02B26/00 |
| Y | WO 2019/002448 A1 (POLIGHT AS [NO]) 3 January 2019 (2019-01-03) * page 9, line 30 - page 14, line 21; figures 1-10 * | 1-16 | |
| Y | EP 2 860 555 A1 (OPTOTUNE AG [CH]) 15 April 2015 (2015-04-15) * paragraph [0088] - paragraph [0089]; figure 7 * * paragraph [0064] - paragraph [0109]; figures 1-12 * | 1-16 | |
| Y | WO 91/17463 A1 (KURTIN STEPHEN [US]) 14 November 1991 (1991-11-14) * page 10, line 3 - page 17, line 10; figures 1-9 * | 1-16 | TECHNICAL FIELDS SEARCHED (IPC) |
| Y | WO 2012/061411 A1 (PIXELOPTICS INC) 10 May 2012 (2012-05-10) * paragraph [0106] - paragraph [0140]; figures 9, 10 * | 1-16 | G02C G02B |
| Y | WO 2017/060537 A2 (OPTOTUNE AG [CH]) 13 April 2017 (2017-04-13) * page 32, line 28 - page 55, line 2; figure 1 * | 1-16 | |
| Y | GB 2 542 638 A (ADIENS LTD [GB]) 29 March 2017 (2017-03-29) * paragraph [0047] - paragraph [0080]; figures 1-7 * | 1-16 | |

-/--

The present search report has been drawn up for all claims

| Place of search | Date of completion of the search | Examiner |
|---|---|---|
| Munich | 20 September 2021 | Bratfisch, Knut |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | Application Number EP 21 17 5484 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 97/19381 A1 (MEDILENS S A R L [FR]; GABRIEL ELIE [FR]) 29 May 1997 (1997-05-29) * page 4, line 3 - page 6, line 27; figures 1-4 * ----- | 4,16 | |
| Y | US 3 246 941 A (MOSS HERBERT L) 19 April 1966 (1966-04-19) * column 3, line 64 - column 4, line 71; figures 1-5 * ----- | 4,16 | |
| | | | TECHNICAL FIELDS SEARCHED (IPC) |

The present search report has been drawn up for all claims

| Place of search | Date of completion of the search | Examiner |
|---|---|---|
| Munich | 20 September 2021 | Bratfisch, Knut |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 21 17 5484

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2021

| Patent document cited in search report | | Publication date | Patent family member(s) | | Publication date |
|---|---|---|---|---|---|
| WO 2018028847 | A1 | 15-02-2018 | CN | 109716215 A | 03-05-2019 |
| | | | EP | 3497507 A1 | 19-06-2019 |
| | | | JP | 2019531500 A | 31-10-2019 |
| | | | US | 2021278703 A1 | 09-09-2021 |
| | | | WO | 2018028847 A1 | 15-02-2018 |
| WO 2019002448 | A1 | 03-01-2019 | AU | 2018294509 A1 | 13-02-2020 |
| | | | BR | 112019027960 A2 | 14-07-2020 |
| | | | CN | 110998374 A | 10-04-2020 |
| | | | CN | 111033321 A | 17-04-2020 |
| | | | EP | 3646067 A1 | 06-05-2020 |
| | | | EP | 3646068 A1 | 06-05-2020 |
| | | | JP | 2020525821 A | 27-08-2020 |
| | | | JP | 2020525822 A | 27-08-2020 |
| | | | KR | 20200018699 A | 19-02-2020 |
| | | | KR | 20200021531 A | 28-02-2020 |
| | | | US | 2020310006 A1 | 01-10-2020 |
| | | | US | 2021141126 A1 | 13-05-2021 |
| | | | WO | 2019002448 A1 | 03-01-2019 |
| | | | WO | 2019002524 A1 | 03-01-2019 |
| EP 2860555 | A1 | 15-04-2015 | EP | 2860555 A1 | 15-04-2015 |
| | | | WO | 2015052233 A1 | 16-04-2015 |
| WO 9117463 | A1 | 14-11-1991 | DE | 4190889 C2 | 30-01-2003 |
| | | | DE | 4190889 T | 18-02-1993 |
| | | | JP | 3072490 B2 | 31-07-2000 |
| | | | JP | H05506944 A | 07-10-1993 |
| | | | US | 5138494 A | 11-08-1992 |
| | | | WO | 9117463 A1 | 14-11-1991 |
| WO 2012061411 | A1 | 10-05-2012 | TW | 201234072 A | 16-08-2012 |
| | | | US | 2012140167 A1 | 07-06-2012 |
| | | | WO | 2012061411 A1 | 10-05-2012 |
| WO 2017060537 | A2 | 13-04-2017 | CN | 108292050 A | 17-07-2018 |
| | | | EP | 3360005 A2 | 15-08-2018 |
| | | | JP | 2018530003 A | 11-10-2018 |
| | | | KR | 20180063886 A | 12-06-2018 |
| | | | US | 2018217402 A1 | 02-08-2018 |
| | | | WO | 2017060537 A2 | 13-04-2017 |
| GB 2542638 | A | 29-03-2017 | BR | 112018006193 A2 | 09-10-2018 |
| | | | CA | 3000067 A1 | 06-04-2017 |
| | | | CN | 108369298 A | 03-08-2018 |
| | | | EP | 3356866 A2 | 08-08-2018 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 21 17 5484

20-09-2021

| Patent document cited in search report | Publication date | Patent family member(s) | Publication date |
|---|---|---|---|
| | | GB 2542638 A | 29-03-2017 |
| | | JP 6831374 B2 | 17-02-2021 |
| | | JP 2018531418 A | 25-10-2018 |
| | | JP 2021009415 A | 28-01-2021 |
| | | KR 20180081054 A | 13-07-2018 |
| | | RU 2018116220 A | 29-10-2019 |
| | | US 2019258084 A1 | 22-08-2019 |
| | | US 2021055574 A1 | 25-02-2021 |
| | | WO 2017055787 A2 | 06-04-2017 |
| WO 9719381 A1 | 29-05-1997 | FR 2741727 A1 | 30-05-1997 |
| | | WO 9719381 A1 | 29-05-1997 |
| US 3246941 A | 19-04-1966 | NONE | |

EPO FORM P0459

page 2 of 2